# EUROPEAN PATENT APPLICATION

(11) **EP 4 830 658 A1**
(43) Date of publication of application: **28.10.2026**
(21) Application number: 24906075.7
(22) Date of filing: 03.12.2024
(51) Int. Cl.: A61B 34/30

(54) **SURGICAL TOOL AND SURGICAL ROBOT SYSTEM**

(30) Priority: 23.12.2023 CN 202311784400; 09.01.2024 CN 202410031137
(71) Applicant: Beijing Surgerii Robotics Company Limited, Beijing 100192 (CN)
(72) Inventor: XU, Kai, Beijing 100192 (CN); ZHAO, Jiangran, Beijing 100192 (CN); DING, Yue, Beijing 100192 (CN); REN, Yitang, Beijing 100192 (CN)
(74) Representative: karo IP
(86) International application number: PCT/CN2024/136325
(87) International publication number: WO 2025/130590

(57) **Abstract**

The present disclosure relates to the field of medical instruments and discloses a surgical tool and a surgical robot system. The tool includes an arm body and a joint mechanism. The arm body includes a distal end, the joint mechanism is disposed at the distal end of the arm body. At least one driving rod sealingly extends from the distal end of the arm body, at least one driving rod is connected to the joint mechanism to drivie the joint mechanism. By forming an integrated sealed structure with the driving rod and the arm body, the interior of the arm body is maintained sealed without affecting movement of the driving rod, and is isolated from the external joint mechanism, preventing bodily fluids from entering the interior of the arm body, thereby facilitating repeated cleaning and sterilization of the surgical tool.

## Description

### Cross-reference to Relevant Applications

This application claims priority to the Chinese patent application filed on December 23, 2023, application number 2023117844001, entitled "Surgical Tool and Surgical Robot System", and Chinese patent application filed on January 9, 2024, application number 2024100311377, entitled "Surgical Tool and Surgical Robot System", the full disclosure of these applications is incorporated herein by reference in their entirety.

### Technical Field

The present disclosure pertains to the field of medical instruments, specifically to a surgical tool and a surgical robot system.

### Background

In minimally invasive medical procedures, surgical tools are commonly used, which include various surgical effectors such as clamps, cutting tools, or needle holders. Surgical effectors are usually mounted at the end of surgical tools and are inserted into small incisions or natural orifices of the patient either directly or through a sheath, and then the corresponding surgical operations are performed.

Conventionally, surgical tools are equipped with driving and transmission mechanisms, such as steel wire pulley assemblies. By connecting the distal end of the steel wire to the surgical operating device and passing the proximal end through the arm body and connecting it to the input end of the motor, the rotational motion input by the motor is converted into linear push-pull motion of the steel wire, thereby controlling the surgical operating device at the end of the surgical tool to perform surgeries on different parts. Since the steel wire rope must extend through the arm body of the surgical tool to connect to the driving motor, the long travel distance tends to result in slack in the wire, affecting drive accuracy.

In addition, the drive and transmission mechanism at the end of conventional surgical tools adopts an open structure exposed to the external environment. This configuration makes cleaning and disinfection difficult, thereby increasing the risk of secondary contamination and infection.

### Summary of the Invention

In some embodiments, the present disclosure provides a surgical tool comprising:
an arm body, comprising a distal end;
a joint mechanism, disposed at a distal end of the arm body; and
at least one driving rod sealingly extending from the distal end of the arm body, wherein the at least one driving rod is connected to the joint mechanism to drive the joint mechanism.

In some embodiments, the present disclosure further provides a surgical robot system comprising:
a mobile station, comprising at least one robotic arm; and
at least one surgical tool as described in any embodiment of the present disclosure, the surgical tool being detachably disposed at a distal end of the robotic arm.

### Brief Description of the Drawings

To describe the technical solutions in the embodiments of the present disclosure more clearly, the accompanying drawings used in the description of the embodiments of the present disclosure are briefly introduced below. The following drawings illustrate only some of the embodiments of the present disclosure. It will be apparent to those of ordinary skill in the art that other embodiments may be obtained based on the contents of these embodiments of the present disclosure and these accompanying drawings without involving any inventive effort.
FIG. 1 illustrates a schematic structural diagram of a surgical tool according to some embodiments of the present disclosure;
FIG. 2 illustrates a side view of a partial structure of a surgical tool according to some embodiments of the present disclosure;
FIG. 3 illustrates a schematic structural diagram of a partial structure of a surgical tool according to some embodiments of the present disclosure;
FIG. 4 illustrates a schematic structural diagram of the connection between the driving rod and the arm body according to some embodiments of the present disclosure;
FIG. 5 illustrates a schematic structural diagram of a driving rod according to some embodiments of the present disclosure;
FIG. 6 illustrates a schematic longitudinal cross-sectional view of a driving rod according to some embodiments of the present disclosure;
FIG. 7 illustrates a schematic diagram of a partial structure of a surgical tool according to some embodiments of the present disclosure;
FIG. 8 illustrates a schematic structural diagram of a wrist joint assembly according to some embodiments of the present disclosure;
FIG. 9 illustrates a schematic structural diagram of a forceps joint assembly according to some embodiments of the present disclosure;
FIG. 10 illustrates a schematic structural diagram of an end instrument and a wrist joint rotation mechanism in one state according to some embodiments of the present disclosure;
FIG. 11 illustrates a schematic structural diagram of an end instrument and a wrist joint rotation mechanism in another state according to some embodiments of the present disclosure;
FIG. 12 illustrates a schematic structural diagram of a slider mating with a loop driving wire according to some embodiments of the present disclosure;
FIG. 13 illustrates a schematic structural diagram of an arm body of a continuum surgical tool according to some embodiments of the present disclosure;
FIG. 14 illustrates a partial structural schematic of a continuum surgical tool according to some embodiments of the present disclosure;
FIG. 15 illustrates a schematic structural diagram of a surgical robot system according to some embodiments of the present disclosure.

### Detailed Description

To make the solved technical problems, used technical solutions, and achieved technical effects of the present disclosure more clearly, the technical solutions of the embodiments of the present disclosure will be further described in detail below with reference to the accompanying drawings. Obviously, the described embodiments are only exemplary embodiments, but not all of embodiments, of the present disclosure.

In the description of the present disclosure, it should be noted that, orientational or positional relationships indicated by the terms "center", "upper", "lower", "left", "right", "vertical", "horizontal", "inner", "outer" and the like are the orientational or positional relationships shown based on the accompanying drawings, and are only for ease of describing the present disclosure and simplifying the description, rather than indicating or implying that the apparatus or element referred to must have a specific orientation or be constructed and operated in a specific orientation, and therefore cannot be construed as limiting the present disclosure. In addition, the terms "first" and "second" are used for descriptive purposes only, and cannot be understood as indicating or implying relative importance. In the description of the present disclosure, it should be noted that, unless otherwise specified and defined, the term "mount", "connected", and "connect", or "couple" should be comprehended in a broad sense. For example, the term may be a fixed connection or a detachable connection; or may be a mechanical connection or an electrical connection; may be a direct connection or an indirect connection via an intermediate medium; or may be internal communication between two elements. For those of ordinary skill in the art, specific meanings of the foregoing terms in the present disclosure may be understood based on specific situations. In the present disclosure, an end close to an operator (e.g., a surgeon) is defined as a proximal end, a proximal portion, a rear end, or a rear portion, and an end close to a patient under surgery is defined as a distal end, a distal portion, a front end, or a front portion. It will be appreciated by those skilled in the art that the embodiments of the present disclosure are applicable to medical instruments or a surgical robots, and may also be applied to other non-medical apparatus.

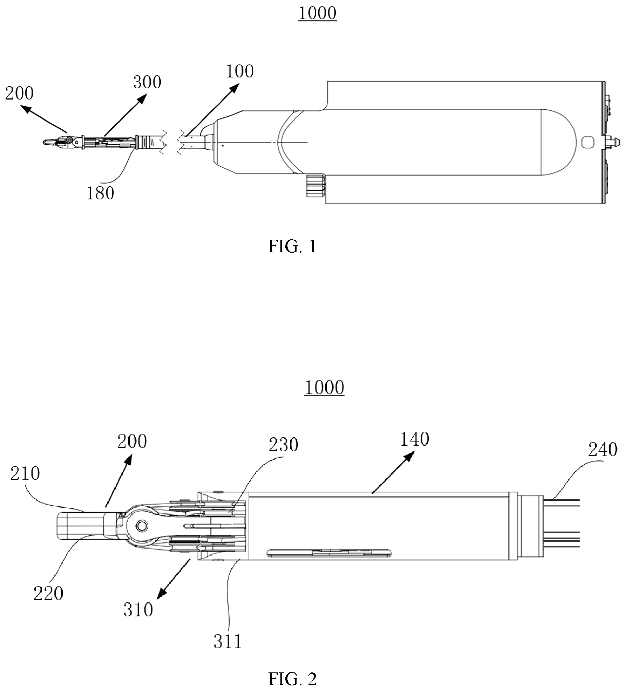

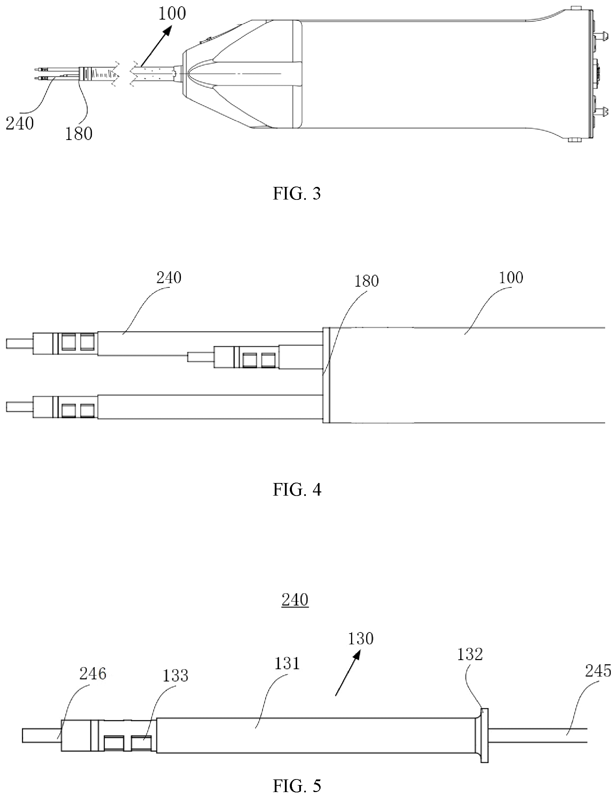

FIG. 1, FIG. 2, and FIG. 3 illustrate schematic structural diagrams and partial schematic structural diagrams of a surgical tool 1000 according to some embodiments of the present disclosure, respectively. For clarity, an arm body 100 is shown in FIG. 2 while a joint mechanism 300 is omitted in FIG. 3. As shown in FIGS. 1-3, the surgical tool 1000 may include an arm body 100, a joint mechanism 300, and at least one driving rod 240. The arm body 100 may include a distal end 180, and the joint mechanism 300 is disposed at the distal end 180 of the arm body 100. The joint mechanism 300 may include any movable joint, such as a hinge joint, a pulley joint, a gear joint, etc. At least one driving rod 240 sealingly extends from the distal end 180 of the arm body 100, and is connected to the joint mechanism 300 to drive the joint mechanism 300.

It should be understood that "sealingly extend" may refer to the driving rod 240 extending from the surface of the distal end 180 of the arm body 100 while maintaining a sealed connection with the distal end 180 during motion. It should be understood that in the present disclosure, the distal end 180 should be interpreted broadly. A sealed connection with the distal end 180 may refer to a direct sealed connection with the surface of the distal end 180 of the arm body 100, or an indirect sealed connection with the distal end 180 of the arm body 100, such as a sealed connection with the surface of other component disposed at the distal end 180 of the arm body 100, for example, a sealed connection may be formed with the surface of a mounting disc disposed at the distal end 180 of the arm body 100, or the inner surface of a bracket 140 (as shown in FIG. 2) disposed at the distal end of the arm body 100. The surface of the distal end 180 may be planar or an uneven surface. In this way, the driving rod 240 and the arm body 100 may form an integrated sealed structure. Without affecting the movement of the driving rod 240, the interior of the arm body 100 is maintained sealed and isolated from the external joint mechanism 300, thereby preventing bodily fluids, bacteria, and viruses from entering the interior of the arm body 100 through pores during surgery, facilitating repeated cleaning and disinfection of surgical tools.

FIG. 4 illustrates a schematic structural diagram of the connection between the driving rod 240 and the arm body 100 according to some embodiments of the present disclosure. In some embodiments, as shown in FIGS. 3 and 4, at least one driving rod 240 may include one or more driving rods. Each driving rod 240 may sealingly extends from the distal end 180 of the arm body 100 respectively. For example, the driving rod 240 may include various driving wires capable of pulling and/or pushing, such as nickel-titanium alloy wires, steel wires, flexible rods, rigid rods, and the like.

In some embodiments, the joint mechanism 300 may include at least one joint assembly. At least one driving rod 240 is connected to the at least one joint assembly, respectively. The at least one driving rod 240 extends through the arm body 100, and a proximal end thereof is configured to receive a pushing or pulling motion to drive the at least one joint assembly to open, close and/or rotate. In some embodiments, the joint mechanism may further include an end instrument 200. The end instrument 200 is disposed at the distal end of the joint mechanism 300. The at least one driving rod 240 may drive the joint mechanism 300 to drive the end instrument 200 to open, close and/or rotate. In some embodiments, as shown in FIG. 1, the arm body 100 may include a distal end and a proximal end, the joint mechanism 300 is disposed at the distal end of the arm body 100. At least one joint assembly is disposed between the end instrument 200 and the distal end of the arm body 100, connected to the end instrument 200, and configured to drive the end instrument 200 to open, close or rotate. It should be understood that the at least one joint assembly may include one or more joint assemblies. By disposing the joint mechanism 300 at the distal end of the arm body 100, the orientation of the end instrument 200 may be adjusted to meet more surgical operation requirements.

It should be understood that the end instrument 200 may include an end effector, an endoscope, or other instruments. The end effector may include, for example, grasping forceps, grasping forceps, scissors, bipolar grasping forceps, monopolar curved scissors, a needle holder, a clip applier, and so on. The endoscope may include, for example, at least one imaging unit and an illumination unit, and so on. Other instruments may include, for example, an electrocautery hook, a drainage tube, or an aspirator, and so on.

In some embodiments, as shown in FIGS. 3 and 4, the at least one driving rod 240 may include a plurality of driving rods, and the at least one joint assembly may include a plurality of joint assemblies. The plurality of driving rods 240 are arranged at intervals, with distal ends sealingly extending from the distal end 180 of the arm body 100, and respectively connected to a plurality of joint assemblies. It should be understood that the arrangement of a plurality of driving rods 240 at intervals may avoid mutual interference, allowing for independent driving of the plurality of joint assemblies. It should also be understood that by providing the plurality of driving rods 240 to drive the plurality of joint assemblies to move, the joint mechanism 300 can achieve multi-degree-of-freedom motion,. For example, the degree of freedom for opening and closing, multiple degrees of freedom for rotation, and so on.

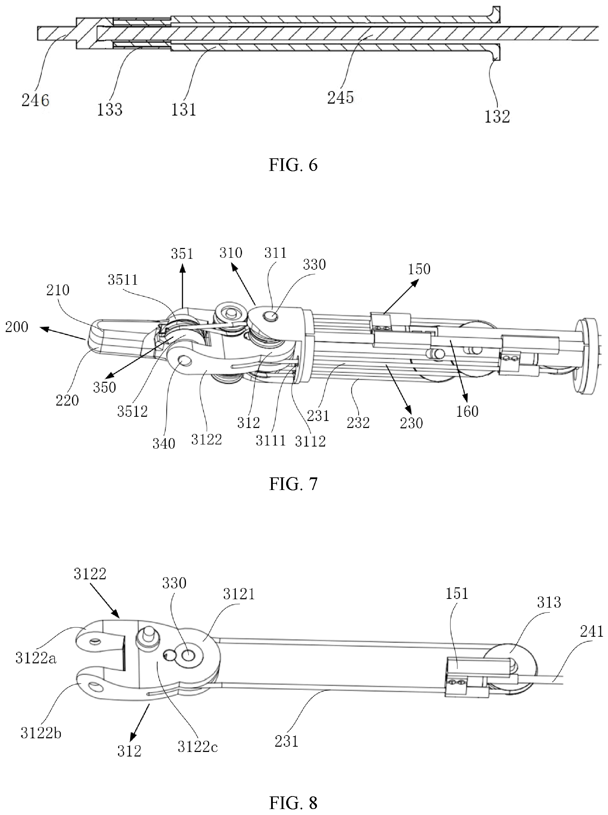

FIGS. 5 and 6 illustrate a schematic structural view and a schematic longitudinal cross-section view of the drive rod 240 according to some embodiments of the present disclosure, respectively. In some embodiments, as shown in FIGS. 5 and 6, the drive rod 240 may include a rod body 245 and a sealing member 130. The distal end of the sealing member 130 is sealingly connected to the distal end of the rod body 245, and the proximal end of the sealing member 130 is sealingly connected to the distal end 180 of the arm body 100, so as to isolate at least a portion of the rod body 245 and the interior of the arm body 100 from the joint mechanism 300. It should be understood that at least a portion of the rod body 245 may include a portion of the rod body 245 enveloped by the sealing member 130. For example, the sealing member 130, the rod body 245, and the arm body 100 form an integrated sealing structure, with the distal end of the rod body 245 extending out of the sealing member 130 to connect with the joint mechanism 300 and configured to drive the unsealed joint mechanism 300.

In some embodiments, as shown in FIGS. 5 and 6, the distal end of the sealing member 130 (e.g., the left side as shown in FIGS. 5 and 6) may be sealingly sleeved on the distal end of the rod body 245 of the drive rod 240, and the proximal end (e.g., the right side as shown in FIGS. 5 and 6) is sealingly disposed at the distal end 180 of the arm body 100. At least a portion of the sealing member 130 is deformable, and the rod body 245 extends through the sealing member 130. In this manner, when the rod body 245 moves (e.g., reciprocates), the sealing member 130 can undergo adaptive deformation, enabling sealing without affecting the movement of the rod body 245. It should be understood that deformability includes, but is not limited to, deformation via the provision of redundant portions or pleated portions, or inherent stretchability of the material itself. At least a portion of the sealing member 130 being deformable may mean that a portion of the sealing member 130 in the axial or radial direction is deformable, or the entire sealing member 130 is deformable. In some embodiments, at least a portion of the sealing member 130 may contain elastically stretchable material, such as rubber or thermoplastic elastomer, or flexible material, such as plastic or nonwoven fabric.

In some embodiments, at least a portion of the sealing member 130 between the distal end and the proximal end is configured to telescopic together with the motion of the rod body 245, thereby isolating the interior of the arm body 100 from the joint mechanism 300. In this manner, it is possible to prevent bodily fluids, bacteria, and viruses from the patient from entering the interior of the arm body 100 during the surgical procedure, facilitating the repeated cleaning and disinfection of surgical tools.

In some embodiments, the sealing member 130 may include an axial portion 131 located at the distal end and a radial portion 132 located at the proximal end. The distal end of the axial portion 131 may be sealingly sleeved at the distal end of the rod body 245, the radial portion 132 is sealingly disposed at the distal end of the arm body 100 in the circumferential direction (e.g., along the circumferential direction of the axial portion 131 or the circumferential direction of the radial portion 132), for example, it may be sealingly connected to the proximal end of the bracket 140. It should be understood that the axial portion 131 and the radial portion 132 may be integrally formed or sealingly connected. The axial portion 131 may be a body portion extending in an axial direction, the radial portion 132 may be a flange portion extending outwardly in the radial direction. In some embodiments, the axial portion 131 of the sealing member 130 may be cylindrical. In some embodiments, a smooth transition section may be provided at a junction of the axial portion 131 and the radial portion 132. By providing the radial portion 132 to increase the contact surface with the distal end of the arm body 100, the sealing connection with the arm body 100 is facilitated. By forming a continuous sealing surface, a better sealing effect can be achieved.

In some embodiments, the axial portion 131 and the radial portion 132 of the sealing member 130 may be integrally formed, and the radial portion 132 may further include a flange located at the proximal end. The flange is used for sealing connection with the distal end of the arm body 100. It should be understood that the longitudinal section of the axial portion 131 and the radial portion 132 may jointly form a shape similar to an "L", "J", or "U".

By providing a deformable sealing member 130, the drive rod 240 and the arm body 100 can form an integrated sealing structure, preventing bodily fluids from entering the interior of the arm body 100. During cleaning and disinfection, the jaw can be repeatedly cleaned and disinfected without disassembling the end of the surgical tool, thereby reducing or avoiding secondary infection caused by bodily fluids entering the interior of the arm body 100. Those skilled in the art may understand that various suitable methods can be used for sealing connection, such as bonding, thermoplastic, clamping, etc.

In some embodiments, as shown in FIG. 5, the drive rod 240 may further include at least one retaining ring 133. The sealing member 130 may have a cylindrical shape. The distal end of the sealing member 130 may be sealingly engaged onto the distal end of the rod body 245, such as on an outer circumferential surface thereof, via the retaining ring 133. It should be understood that the proximal end of the sealing member 130 can be sealingly connected to the distal end of the arm body 100 by means of bonding, thermo welding, clamping, or the like. Providing the retaining ring 133 can further ensure the sealing between the sealing member 130 and the rod body 245. The risk of the sealing member 130 slipping during telescopic motion is reduced.

FIG. 7 illustrates a partial schematic structural diagram of a surgical tool 1000 according to some embodiments of the present disclosure. In FIG. 7, for clarity, the bracket 140 is partially hidden. In some embodiments, as shown in FIG. 7, the joint assembly may include at least one loop driving wire 230. At least one loop driving wire 230 forms at least one loop on at least one joint assembly, respectively. The distal end of at least one driving rod 240 is connected to at least one loop driving wire 230, the proximal end passes through the arm body 100, used for pushing or pulling the at least one loop driving wire 230. It should be understood that the loop driving wire 230 may include a driving wire that forms a closed loop, such as a nickel-titanium alloy wire, steel wire rope, cable, belt structure, chain structure, etc.. The loop driving wire 230 may be distributed along the joint assembly and wound around the joint assembly. In some embodiments, the driving rod 240 may drive the wire by a rod-like or tubular structure made of nickel-titanium alloy wire. For example, the quantity of joint assemblies may be one or more, the quantity of loop driving wires 230 may be one or more, and the quantity of driving rods 240 may be one or more. It should be understood that the quantity of joint assemblies may be consistent with the quantity of loop driving wires 230 and driving rods 240, or one joint assembly may include a plurality of loop driving wires 230.

By connecting the driving rod through the arm body to the driving device (such as a motor), the issue of slack caused by the long travel distance of steel cables extending through the arm body is avoided. Driving with one nickel-titanium alloy wire instead of driving two steel cables simplifies the driving structure and enhances driving accuracy.

In some embodiments, as shown in FIG. 2, the joint assembly may further comprise a bracket 140. The bracket 140 is fixedly disposed at the distal end of the arm body 100, and the end instrument 200 is disposed at the distal end of the bracket 140. It should be understood that the bracket 140 may be composed of a plurality of link rods or plate-like structures (such as a distal plate, a proximal plate, or a side plate, etc.), to accommodate or support the joint mechanism 300.

FIG. 8 illustrates a schematic structural diagram of a wrist joint assembly 310 according to some embodiments of the present disclosure. In some embodiments, as shown in FIGS. 7 and 8, at least one joint assembly may include at least one wrist joint assembly 310. The at least one loop driving wire 230 of the wrist joint assembly 310 may include a loop wrist joint driving wire 231. The at least one driving rod 240 may include a wrist joint driving rod 241.

As shown in FIG. 7, the wrist joint assembly 310 may include a wrist joint base 311 and a wrist joint rotation mechanism 312. The wrist joint base 311 is fixedly disposed at the distal end of the bracket 140. The wrist joint rotation mechanism 312 is rotatably disposed on the wrist joint base 311 and connected to the loop wrist joint driving wire 231, for rotating about a first axis (e.g., an axis parallel to the first pin 330) when the wrist joint driving rod 241 drives the loop wrist joint driving wire 231. For example, the wrist joint rotation mechanism 312 may be pivotally connected to the wrist joint base 311 via the first pin 330. The first axis may be a rotational axis along a lengthwise direction of the first pin 330. The wrist joint driving rod 241 is configured to push or pull the loop wrist joint driving wire 231 under the received drive, so as to drive the wrist joint rotation mechanism 312 through the loop wrist joint driving wire 231 to rotate about the first axis in a plane perpendicular to the first axis. It should be understood that one or more wrist joint assemblies 310 may be provided, and the plurality of wrist joint assemblies 310 may move independently of each other. For example, the plurality of wrist joint assemblies 310 may include two independent wrist joint assemblies with rotation axes perpendicular to each other. By providing the wrist joint assembly 310, articulation of the end instrument 200 of the surgical tool may be achieved, thereby increasing the flexibility of movement.

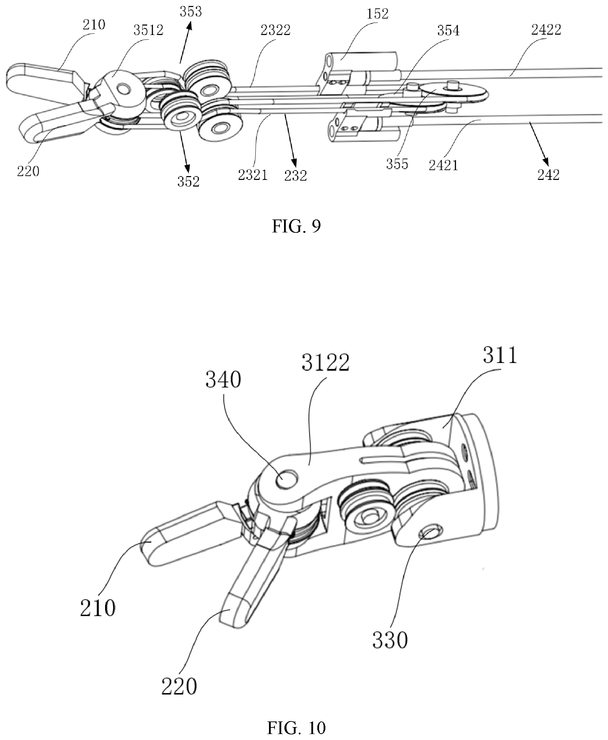

FIG. 9 illustrates a schematic structural diagram of a forceps joint assembly 350 according to some embodiments of the present disclosure. In some embodiments, as shown in FIGS. 7 and 9, at least one joint assembly may include the forceps joint assembly 350. The end instrument 200 may include a first jaw 210 and a second jaw 220. At least one loop driving wire 230 of the forceps joint assembly 350 may include at least one loop forceps joint driving wire 232, at least one driving rod 240 may include at least one forceps joint driving rod 242.

As shown in FIGS. 7 and 9, the forceps joint assembly 350 may further include a forceps joint opening-and-closing mechanism 351. The forceps joint opening-and-closing mechanism 351 is disposed on the wrist joint rotation mechanism 312 and is connected to at least one loop forceps joint driving wire 232. The first jaw 210 and the second jaw 220 are disposed on the forceps joint opening-and-closing mechanism 351. The forceps joint opening-and-closing mechanism 351 is utilized to rotate with the wrist joint rotation mechanism 312 and to drive the first jaw 210 and/or the second jaw 220 to open and close about a second axis (e.g., an axis parallel to the second pin 340) when at least one forceps joint driving rod 242 drives at least one loop forceps joint driving wire 232. It should be understood that the forceps joint opening-and-closing mechanism 351 may be pivotally connected to the wrist joint rotation mechanism 312 via the second pin 340, the second axis may be a rotational axis along a lengthwise direction of the second pin 340. For example, the forceps joint driving rod 242 is configured to push or pull the loop forceps joint driving wire 232 under the received drive, so as to drive the forceps joint opening-and-closing mechanism 351 to rotate about the second axis within a plane perpendicular to the second axis via the loop forceps joint driving wire 232, thereby driving the first jaw 210 and/or the second jaw 220 to open and close within a plane perpendicular to the second axis. In some embodiments, the second axis may be perpendicular to the first axis.

It should be understood that the motion of the forceps joint opening-and-closing mechanism 351 and the motion of the wrist joint rotation mechanism 312 may be controlled independently or cooperatively. For example, when the wrist joint rotation mechanism 312 rotates, the forceps joint opening-and-closing mechanism 351 may drive the opening and closing of the first jaw 210 and/or the second jaw 220. Alternatively, when the wrist joint rotation mechanism 312 does not rotate, the forceps joint opening-and-closing mechanism 351 may drive the opening and closing of the first jaw 210 and/or the second jaw 220. It should be understood that in this disclosure, the jaw should be interpreted broadly to include any tool head of an opening and closing tool, including but not limited to pliers-type tools, scissor-type tools, etc. Those skilled in the art will understand that in some embodiments, the end instrument 200 is not an opening and closing instrument, such as an endoscope, an electrosurgical hook, etc., and at least one joint assembly may not include the forceps joint assembly 350.

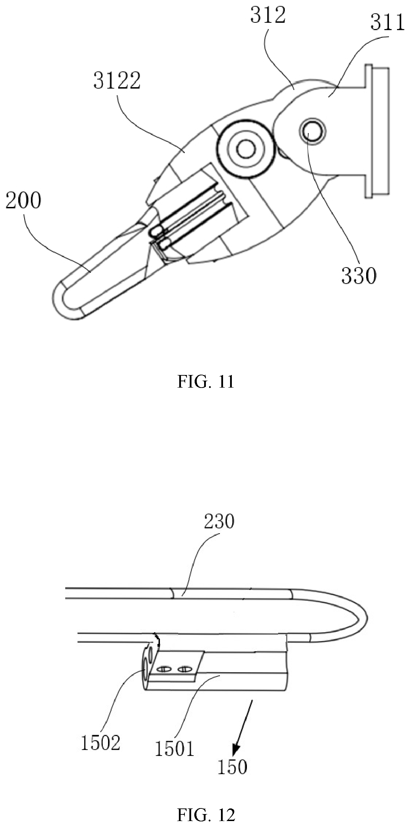

FIGS. 10 and 11 illustrate schematic structural diagrams of an end instrument 200 and a wrist joint rotation mechanism 312 in various states according to some embodiments of the present disclosure, respectively. For simplicity, the loop driving wire 230 is omitted in FIGS. 10 and 11. In FIG. 10, the first jaw 210 and the second jaw 220 of the end instrument 200 are in an open state, and the wrist joint rotation mechanism 312 is in a non-rotating state (for example, the central longitudinal axis of the jaw base 3122 is substantially aligned on the same axis as the central longitudinal axis of the wrist joint base 311). In FIG. 11, the first jaw 210 and the second jaw 220 of the end instrument 200 are in a closed state, the wrist joint rotation mechanism 312 is in a rotating state (for example, the central longitudinal axis of the jaw base 3122 forms an angle with the central longitudinal axis of the wrist joint base 311). The foregoing are merely examples, and the forceps joint opening-and-closing mechanism 351 and the wrist joint rotation mechanism 312 may be controlled according to actual needs to achieve operations in various states.

In some embodiments, as shown in FIGS. 7 and 8, the wrist joint rotation mechanism 312 may include a wrist joint rotation body 3121 and a jaw base 3122. The wrist joint rotation body 3121 is rotatably connected to the wrist joint base 311 (e.g., via a pivot connection). The jaw base 3122 is disposed on the wrist joint rotation body 3121. The wrist joint rotation body 3121 may include a wheel that can rotate about a first axis, and the jaw base 3122 is fixedly connected to the wheel so as to rotate along with the wheel.

In some embodiments, as shown in FIG. 8, the jaw base 3122 may include a first jaw bracket 3122a and a second jaw bracket 3122b. The first jaw bracket 3122a and the second jaw bracket 3122b may be symmetrically disposed on the wrist joint rotation body 3121. For example, the first jaw bracket 3122a and the second jaw bracket 3122b may extend distally in a direction perpendicular to the plane containing the rotation axis (e.g., the first axis) of the wrist joint rotation body 3121. A receiving space is formed between the first jaw bracket 3122a and the second jaw bracket 3122b, which can be used to accommodate the forceps joint opening-and-closing mechanism 351.

In some embodiments, as shown in FIG. 8, the jaw base 3122 may further include a connecting plate 3122c. The first effector bracket 3122a and the second effector bracket 3122b are symmetrically arranged. The connecting plate 3122c is fixedly disposed between the first jaw bracket 3122a and the second jaw bracket 3122b, connecting the first jaw bracket 3922a and the second jaw bracket 3922b together. The connecting plate 3122c is integrally formed with or fixedly connected to the wrist joint rotation body 3121.

In some embodiments, as shown in FIG. 7, the wrist joint base 311 located on both sides of the wrist joint rotation body 3121 is provided with a loop wrist joint driving wire guide hole 3111 and a loop forceps joint driving wire guide hole 3112, respectively. The loop wrist joint driving wire 231 and the loop forceps joint driving wire 232 may pass through the loop wrist joint driving wire guide hole 3111 and the loop forceps joint driving wire guide hole 3112, respectively.

In some embodiments, as shown in FIG. 8, the wrist joint assembly 310 may further include a wrist fixed pulley 313. The wrist fixed pulley 313 is rotatably disposed on the bracket 140 (e.g., connected via a pivot). The distal end of the loop wrist joint driving wire 231 is wound around the wrist joint rotation body 3121, the proximal end thereof is wound around the wrist fixed pulley 313. It should be understood that the wrist joint rotation body 3121 and the wrist fixed pulley 313 may include wire grooves disposed circumferentially. The distal end of the loop wrist joint driving wire 231 is wound in the wire groove of the wrist joint rotation body 3121, the proximal end thereof is wound in the wire groove of the wrist fixed pulley 313, so as to form a closed loop. The wrist joint driving rod 241 may be connected to the loop wrist joint driving wire 231 between the distal and proximal ends of the loop wrist joint driving wire 231. The wrist joint driving rod 241 pushes or pulls the loop wrist joint driving wire 231, driving reciprocal motion of the loop wrist joint driving wire 231 within the closed loop, thereby driving the wrist joint rotation body 3121 to rotate in forward and reverse directions, and further driving the jaw base 3122 to perform rotational motion in a plane perpendicular to the rotational axis of the wrist joint rotation body 3121.

In some embodiments, as shown in FIGS. 7 and 9, the forceps joint opening-and-closing mechanism 351 may include at least one forceps rotation body (e.g., the first forceps rotation body 3511 and the second forceps rotation body 3512 shown in FIG. 7). At least one forceps rotation body is fixedly connected to the first jaw 210 and/or the second jaw 220 and rotatably connected to the jaw base 3122. The forceps joint assembly 350 may further include a first set of steering pulleys 352, a second set of steering pulleys 353, and at least one forceps fixed pulley (e.g., the first forceps fixed pulley 354 and the second forceps fixed pulley 355). The first set of steering pulleys 352 and the second set of steering pulleys 353 are rotatably connected to the jaw base 3122 respectively, for example, via a pivot connection. It should be understood that the forceps rotation body may include a wheel. The forceps rotation body may be fixedly connected to the jaw or integrally formed with the jaw. At least one forceps fixed pulley is rotatably disposed on the bracket 140, and the distal end of at least one loop forceps joint driving wire 232 is wound around at least one forceps rotation bodywhile bypassing the first set of steering pulleys 352 and the second set of steering pulleys 353, while the proximal end is wound around at least one forceps fixed pulley. By providing multiple sets of steering pulleys, the direction of rotation of the loop forceps joint driving wire 232 may be changed to alter the rotational direction of the forceps rotation body.

In some embodiments, as shown in FIGS. 7-9, the first set of steering pulleys 352 and the second set of steering pulleys 353 may be rotatably connected to the connecting plate 3122c. The first set of steering pulleys 352 and the second set of steering pulleys 353 may be symmetrically disposed on both sides of the connecting plate 3122c. In some embodiments, at least one forceps rotation body may include one forceps rotation body, which is connected to one of the first jaw 210 and the second jaw 220, the other one of the first jaw 210 and the second jaw 220 is fixedly disposed on the jaw base 3122. At least one loop forceps joint driving wire 232 includes one loop forceps joint driving wire, and at least one forceps fixed pulley includes one forceps fixed pulley. In this way, one jaw can be driven to open and close relative to the other jaw.

In some embodiments, as shown in FIGS. 7-9, the pulleys located at the proximal end of the first set of steering pulleys 352 and the second set of steering pulleys 353 may be coaxially arranged with the wrist joint rotation mechanism 312. For example, the pulleys located at the proximal end of the first set of steering pulleys 352 and the second set of steering pulleys 353 may be pivotally connected to the wrist joint base 311 via a first pin 330. This can simplify the structure of the joint mechanism and make the structure of the end of the surgical tool more compact.

In some embodiments, as shown in FIGS. 7 and 9, at least one forceps rotation body may include a first forceps rotation body 3511 and a second forceps rotation body 3512. The first forceps rotation body 3511 is fixedly connected to the first jaw 210 and rotatably connected to the jaw base 3122, the second forceps rotation body 3512 is fixedly connected to the second jaw 220 and rotatably connected to the jaw base 3122. At least one forceps fixed pulley includes a first forceps fixed pulley 354 and a second forceps fixed pulley 355, which are rotatably disposed on the bracket 140, respectively. At least one forceps joint driving rod 242 may include a first forceps joint driving rod 2421 and a second forceps joint driving rod 2422. At least one loop forceps joint driving wire 232 includes a first loop forceps joint driving wire 2321 and a second loop forceps joint driving wire 2322. The first forceps joint driving rod 2421 may be connected to the first loop forceps joint driving wire 2321 to drivve the first loop forceps joint driving wire 2321 to reciprocate, the second forceps joint driving rod 2422 may be connected to the second loop forceps joint driving wire 2322 to drive the second loop forceps joint driving wire 2322 to reciprocate. The distal end of the first loop forceps joint driving wire 2321 is wound around the first forceps rotation body 3511while bypassing the first set of steering pulleys 352 and the second set of steering pulleys 353, the proximal end is wound around the first forceps fixed pulley 354. The distal end of the second loop forceps joint driving wire 2322 is wound around the second forceps rotation body 3512while bypassing the first set of steering pulleys 352 and the second set of steering pulleys 353, the proximal end is wound around the second forceps fixed pulley 355. It should be understood that the structure of the forceps fixed pulley may be similar to that of the wrist fixed pulley. In some embodiments, the first forceps fixed pulley 354 and the second forceps fixed pulley 355 may be located at different positions along the axis of the bracket 140. It should be understood that the rotational axes of the first forceps rotation body 3511 and the second forceps rotation body 3512 are coaxial or parallel. By setting up two forceps rotation bodies, it is possible to drive the opening and closing of two jaws.

FIG. 12 illustrates a schematic structural diagram of the mating between a slider 150 and a loop driving wire 230 according to some embodiments of the present disclosure. In some embodiments, as shown in FIGS. 7 and 12, the joint mechanism 300 may further include at least one slider 150 and at least one guiding mechanism 160. At least one slider 150 is fixedly connected to at least one loop driving wire 230, respectively. At least one guiding mechanism 160 is fixedly disposed along an axial direction of the distal end of the arm body 100 between the end instrument 200 and the distal end of the arm body 100. The slider 150 is slidably connected to the guiding mechanism 160. The slider 150 is configured to perform linear motion along the guide mechanism 160 under the drive of the drive rod 240. For example, as shown in FIG. 7, at least one guiding mechanism 160 may be disposed within a bracket 140 and fixedly connected to the bracket 140.

In some embodiments, the guiding mechanism 160 may include a guide rod. The slider 150 is provided with a corresponding through hole 1502. The guide rod passes through the corresponding through hole 1502, allowing the slider 150 to move linearly along the guide rod. In some embodiments, the guiding mechanism 160 may include a guide rail. The slider 150 is provided with a corresponding sliding slot. The slider 150 is slidably disposed on the guide rail through the sliding slot, allowing the slider 150 to move linearly along the guide rail. The above is merely an example and is not limited thereto. The guiding mechanism 160 may also include any other structure capable of achieving guidance. It should be understood that the quantity of sliders 150 may be consistent with the quantity of drive rods 240, the quantity of guiding mechanisms 160 may be consistent or inconsistent with the quantity of sliders 150.

In some embodiments, as shown in FIGS. 8 and 9, at least one slider 150 may include a wrist joint slider 151 and at least one forceps joint slider 152. The wrist joint slider 151 may be fixedly connected to a loop wrist joint driving wire 231. At least one forceps joint slider 152 may be fixedly connected to at least one loop forceps joint driving wire 232, respectively. The distal end of a wrist joint driving rod 241 is fixedly connected to the wrist joint slider 151. The distal end of at least one forceps joint driving rod 242 is fixedly connected to at least one forceps joint slider 152, respectively. The proximal ends of the wrist joint driving rod 241 and at least one forceps joint driving rod 242 are configured to receive push or pull drive, so as to drive the wrist joint slider 151 and at least one forceps joint slider 152, respectively, to reciprocate linearly along the corresponding guide mechanism 160. In some embodiments, the wrist joint slider 151 and the forceps joint slider 152 (e.g., one of the forceps joint sliders 152) may be disposed on the same guide mechanism 160, as shown in FIG. 7, thereby reducing the quantity of guide mechanisms, simplify the structure, and facilitate the miniaturization and lightweight of surgical tools.

As shown in FIG. 12, the slider 150 may include a main body 1501, as well as a through hole 1502 and a connecting hole (not shown) disposed on the main body 1501. As shown in FIG 6, the distal end of the driving rod 240 may include a connecting head 246 for mating with the connecting hole. The proximal end of the connecting head 246 may be fixedly connected to the distal end of the rod body 245 of the driving rod 240, such as by clamping or welding. The distal end of the connecting head 246 may be fixedly connected to the connecting hole. The main body 1501 of the slider 150 may be fixedly connected to the loop driving wire 230. By providing the connecting hole and the connecting head 246, the driving rod 240 can be processed and assembled more easily.

In some embodiments, the end instrument 200 may be scissors. The proximal end of the at least one driving rod 240 (for example, at least one forceps joint driving rod 242 or wrist joint driving rod 241) may be connected to a power supply apparatus (such as a monopolar energy generator). The at least one driving rod 240, at least a portion of the joint mechanism 300, and the end instrument 200 form a conductive path to create a monopolar surgical tool, facilitating monopolar operations, so as to perform operations such as electric shearing and cutting. It should be understood that at least a portion of the joint mechanism 300 may be a conductive part, and the joint mechanism 300 may also include an insulating part.

In some embodiments, the end instrument 200 may be a clamp tool (e.g., including a first jaw 210 and a second jaw 220). The driving rod 240 may include a plurality of driving rods (e.g., a first forceps joint driving rod 2421 and a second forceps joint driving rod 2422). The first forceps joint driving rod 2421 and the second forceps joint driving rod 2422 are connected to a power supply apparatus, respectively (e.g., the proximal end of the first forceps joint driving rod 2421 and the proximal end of the second forceps joint driving rod 2422 are used to connect to a bipolar energy generator, respectively). And the first forceps joint driving rod 2421 and the second forceps joint driving rod 2422 are insulated from each other. The first forceps joint driving rod 2421 forms a first conductive path with the first jaw 210, the second forceps joint driving rod 2422 forms a second conductive path with the second jaw 220. The first conductive path and the second conductive path are insulated from each other to form a bipolar surgical tool, facilitating bipolar operations, so as to perform operations such as electrocoagulation.

In some embodiments, the proximal end of the first forceps joint driving rod 2421 is used to connect a monopolar energy generator and a bipolar energy generator, respectively. The proximal end of the second forceps joint driving rod 2422 is used to connect a bipolar energy generator. The first jaw 210 and the second jaw 220 of the end instrument 200 form a first conductive path and a second conductive path, respectively, which are insulated from each other, with the first forceps joint driving rod 2421 and the second forceps joint driving rod 2422, to achieve monopolar or bipolar operations. In this way, monopolar or bipolar operations can be selectively performed on the same surgical tool. It should be understood that in this disclosure, the jaw of the end instrument 200 should be broadly interpreted, and may include the tool head of any opening and closing tool, including but not limited to pliers-type tools, scissor-type tools, and the like.

Those skilled in the art should understand that in the case where a driving rod (such as the forceps joint driving rod 242) is connected to a power supply apparatus (for example, in the case where the forceps joint driving rod 242 forms a conductive path with the end instrument 200), an insulating member may be provided at an appropriate position of the surgical tool (such as at least a portion of the structures of the wrist joint rotation mechanism 312, or between the wrist joint rotation mechanism 312 and the end instrument 200) to prevent the non-operating portion of the surgical tool (for example, the outer circumference of the wrist joint rotation mechanism 312) from being charged, which may cause burns to healthy tissues, thereby ensuring the end instrument 200 to perform unipolar or bipolar operations normally. It should be understood that the insulating member may be an individual structural component or a portion of the wrist joint rotation mechanism 312.

In some embodiments, the distal end of the drive rod 240 may include an insulating coating, the insulating coating is configured to insulate the drive rod 240 from the end instrument 200. It should be understood that the proximal end of the drive rod 240 may be energized. The insulating coating insulates the distal end of the drive rod 240 from the slider 150 to prevent the slider 150 from conducting electricity, thereby avoiding the formation of a path between the end instrument 200 and the drive rod 240 through the slider 150. It should be understood that an insulating member can also be provided between the distal end of the arm body 100 and the joint mechanism 300 to insulate the arm body 100 from the end instrument 200. In this way, a passive surgical tool, such as a non-charged tissue grasper, can be formed.

In some embodiments, the arm body 100 may include a rigid-segment arm body. Alternatively, the arm body 100 may include a deformable arm body. For example, the arm body 100 may include a rigid arm body located at the proximal end and a deformable arm body located at the distal end. It should be understood that the deformable arm body may include an articulated arm body (such as snake bone structure arm body), a flexible arm body (such as flexible tube), continuum arm body, and the like.

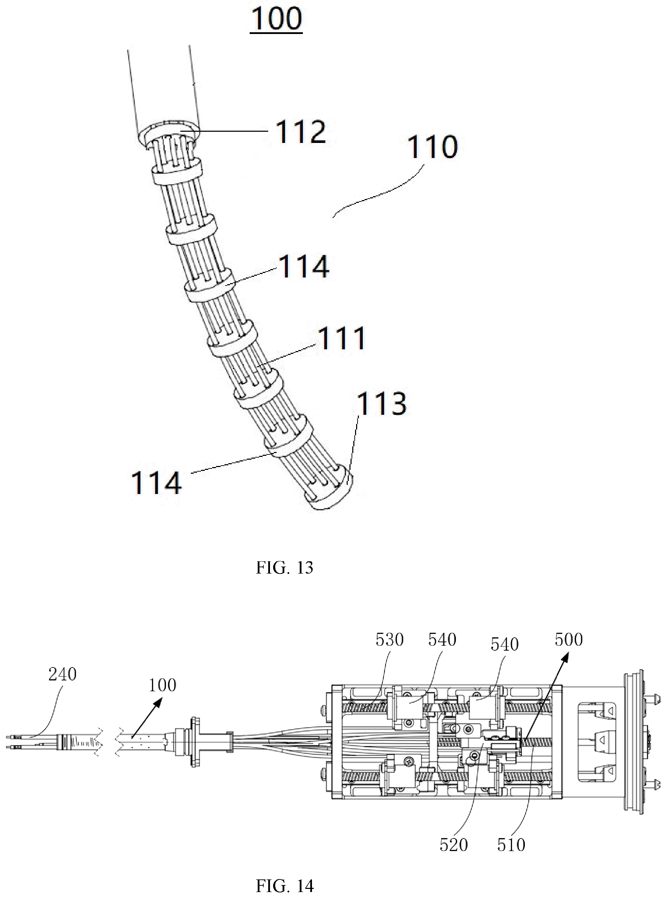

FIG. 13 illustrates a schematic structural diagram of an arm body of a continuum surgical tool according to some embodiments of the present disclosure. In some embodiments, the arm body 100 of the continuum surgical tool may include a continuum structure arm body. For simplicity of explanation, the arm body 100 is only shown as a schematic structural diagram including one distal continuum segment 110. As shown in FIG. 13, the continuum structure arm body may include at least one distal continuum segment 110, which may include a plurality of distal structural backbones 111, a distal basal disk 112, a distal stopping disk 113, and at least one distal spacer disk 114 disposed between the distal basal disk 112 and the distal stopping disk 113. The distal ends of the plurality of distal structural backbones 111 are fixedly connected to the distal stopping disk 113. The plurality of distal structural backbones 111 slidably pass through at least one distal spacer disk 114 and the distal basal disk 112. The proximal ends of the plurality of distal structural backbones 111 are configured to receive a push or pull drive to drive the movement of the distal continuum segment 110. It should be understood that a plurality of distal structural backbones 111 may be distributed at radially opposite positions, the bending of the distal continuum segment 110 can be driven by cooperatively pushing or pulling two structural backbones which are positioned opposite to each other. It should be understood that the quantity of structural backbones is not limited here and can be adjusted according to the actual load that the surgical tool needs to bear. The reliability and load capacity of the arm body 100 of the surgical tool 1000 can be improved through the continuum structure arm body. For example, the event of a fracture of one structural backbone does not affect the motion of the arm body 100, thereby enhancing the safety of the surgical tool 1000.

In some embodiments, the distal stopping disk 113 may be fixedly connected to the proximal end of the joint mechanism 300 of the surgical tool 1000. For example, the distal basal disk 112, at least one distal spacer disk 114, and the distal stopping disk 113 may be arranged at intervals, with corresponding through holes distributed circumferentially on each disk. A plurality of distal structural backbones 111 may slide through the through holes on the distal spacer disks 114 and the distal basal disk 112. In some embodiments, the shapes of the distal basal disk 112, the distal stopping disk 113, and the distal spacer disks 114 may be ring-shaped structures, disk-shaped structures, or other suitable structures, and the cross-sections may be circular, rectangular, polygonal, or various other shapes. A plurality of distal spacer disks 114 are arranged at intervals to enhance the stability of the plurality of distal structural backbones 111 when being pushed or pulled. It should be understood that the distal structural backbones 111 may include elastic thin rods or elastic thin tubes that are made of super-elastic materials, such as nickel-titanium alloy materials. Those skilled in the art will understand that the quantity of distal spacer disks 114 included in the distal continuum segment 110 may be any suitable number, and is not limited herein. It should be understood that the quantity of the distal continuum segments 110 may also be two or more, and by driving a plurality of distal continuum segments 110 to bend, the arm body may be bended in multiple degrees of freedom, thereby increasing the flexibility of the arm body.

It should be understood that the distal continuum segment 110 may be deformed by pushing or pulling the distal structural backbone 111 with a driving device. For example, the driving device may bend the distal continuum segment 110 by driving the distal structural backbone 111. In some embodiments, the driving device may include a linear motion mechanism, a driving segment (such as a proximal continuum segment), or a combination of both. The linear motion mechanism may be connected to the distal structural backbone 111 of one or more distal continuum segments 110 to push or pull the distal structural backbone 111, thereby driving the bending of one or more distal continuum segments 110. The driving segment may include a fixed disk and a plurality of proximal structural backbones, where one end of the plurality of proximal structural backbones is fixedly connected to the fixed disk. The other end of the plurality of proximal structural backbones of the driving segment is connected to or integrally formed with the plurality of distal structural backbones 111, so as to bend the distal continuum segment 110 through the bending of the driving segment.

FIG. 14 illustrates a partial structural schematic of a continuum surgical tool according to some embodiments of the present disclosure. In some embodiments, as shown in FIG. 14, the surgical tool 1000 may further include at least one transmission mechanism 500. The at least one transmission mechanism 500 is connected to the proximal ends of the at least one driving rod 240, respectively, for pushing or pulling the at least one driving rod 240. In some embodiments, the transmission mechanism 500 may include a screw 510 and a screw nut 520 connected to the screw 510. The screw 510 is configured to be connected to with the motor of the driving device, and the screw nut 520 is connected with the proximal end of the driving rod 240. By driving the screw 510 to rotate with a motor, the screw nut 520 may be driven to move linearly along the screw 510, thereby pushing or pulling the drive rod 240. It should be understood that the quantity of transmission mechanisms may be adjusted according to the quantity of driving rods 240.

In some embodiments, as shown in FIG. 14, the transmission mechanism 500 may further comprise a double-headed screw 530 and a pair of screw nuts 540 connected to the double-headed screw 530. The double-headed screw 530 may be connected to the screw nuts 540, respectively. The screw nuts 540 may be connected to the distal structural backbone 111 of the distal continuum segment 110 or the proximal structural backbone of the driving segment. When the double-headed screw 530 is driven to rotate, the screw nuts 540 can be driven to move linearly in opposite directions at the same velocity, thereby achieving coordinated pushing and pulling of the two structural backbones to drive the bending of the distal continuum segment 110. It should be understood that the plurality of transmission mechanisms 500 may be combinations of screws and double-headed screws. The foregoing are merely examples. It should be understood that the transmission mechanism may include any mechanism capable of achieving linear motion.

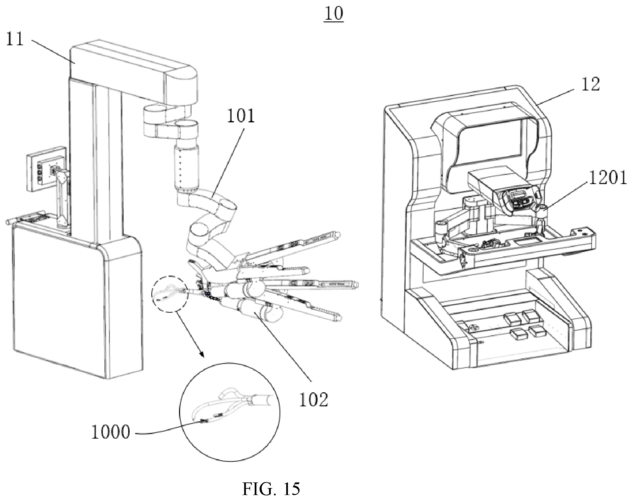

Some embodiments of the present disclosure also provide a surgical robot system. FIG. 15 illustrates a schematic structural diagram of a surgical robot system 10 according to some embodiments of the present disclosure. As shown in FIG. 15, the surgical robot system 10 may include a mobile station 11 and a surgical tool (e.g., surgical tool 1000) as in any of the embodiments of the present disclosure. The mobile station 11 may include at least one robotic arm 101, the surgical tool 1000 may be detachably disposed at the distal end of the robotic arm 101. It should be understood that the robotic arm 101 may include a plurality of movable joints and links, having multiple degrees of freedom. The surgical tool (e.g., surgical tool 1000) may be detachably disposed at the distal end of the robotic arm 101, the robotic arm 101 is used to adjust the position and orientation of the surgical tool end (e.g., end instrument 200).

In some embodiments, the surgical robot system 10 may further include a surgeon console 12. The mobile station 11 and the surgeon console 12 may be connected via wired transmission or wireless transmission. During surgery, the user controls the surgical tools and/or imaging tools (such as endoscopes) included in the mobile station 11 to perform operations by operating the master manipulator 1201 included in the surgeon console 12. The mobile station 11 is typically located on the side of the patient. In response to the control instructions from the surgeon console 12, the mobile station 11 performs surgical operations on the patient. In some embodiments, the user may also control the opening and closing of the jaws (such as the first jaw 210 and/or the second jaw 220) of the surgical tool 1000 or control the rotation of the wrist assembly 310 by operating the master manipulator 1201, thereby driving the motion of the end instrument 200 of the surgical tool 1000.

In some embodiments, the mobile station 11 of the surgical robot system 10 may further comprise at least one driving device 102. The at least one driving device 102 may be disposed between at least one surgical tool (such as surgical tool 1000) and at least one robotic arm 101. As shown in FIG. 15, the mobile station 11 may comprise a single robotic arm 101. A plurality of driving devices 102 may be disposed on the robotic arm 101. Those skilled in the art will understand that the mobile station 11 may also comprise a plurality of robotic arms, each robotic arm may be equipped with one or more driving devices, which is not specifically limited herein. Those skilled in the art will understand that the surgical robot 10 provided in this embodiment may be any suitable surgical robot, including endoscopic surgical robots.

This disclosure also discloses the following embodiments:
Item 1: a surgical tool, comprising:
   an arm body, the arm body includes a distal end and a proximal end;
   a joint mechanism disposed at a distal end of the arm body;
   the joint mechanism includes at least one joint assembly, the joint assembly includes at least one loop driving wire, the at least one loop driving wire forms at least one loop on the joint assembly, respectively; and
   at least one driving rod, the distal end of the at least one driving rod is connected to the at least one loop driving wire respectively, and the at least one driving rod passes through the arm body, the proximal end thereof is configured to receive a drive to push or pull the at least one loop driving wire, thereby driving the at least one joint assembly.
Item 2: the surgical tool according to Item 1, wherein the joint mechanism further includes:
   a bracket fixedly disposed at the distal end of the arm body;
   the at least one joint assembly includes at least one wrist joint assembly, wherein at least one loop driving wire of the wrist joint assembly includes a loop wrist joint driving wire, the at least one driving rod includes a wrist joint driving rod, the wrist joint assembly further includes:
      a wrist joint base fixedly disposed at the distal end of the bracket;
      a wrist joint rotation mechanism rotatably disposed on the wrist joint base and connected to the loop wrist joint driving wire, and is configured to rotate about a first axis when the wrist joint driving rod drives the loop wrist joint driving wire.
Item 3: the surgical tool according to Item 2, wherein the joint mechanism further includes:
   an end instrument disposed at the distal end of the joint mechanism, the end instrument includes a first jaw and a second jaw;
   the at least one joint assembly includes a forceps joint assembly, at least one loop driving wire of the forceps joint assembly includes at least one loop forceps joint driving wire, the at least one driving rod includes at least one forceps joint driving rod, the forceps joint assembly further includes:
      a forceps joint opening-and-closing mechanism disposed on the wrist joint rotation mechanism and connected to the at least one loop forceps joint driving wire, wherein the first jaw and the second jaw are disposed on the forceps joint opening-and-closing mechanism, the forceps joint opening-and-closing mechanism is configured to rotate together with the wrist joint rotation mechanism and is configured to drive the first jaw and/or the second jaw to open and close around a second axis when the at least one forceps joint driving rod drives the at least one loop forceps joint driving wire.
Item 4: the surgical tool according to Item 3, wherein the wrist joint rotation mechanism further includes:
   a wrist joint rotation body rotatably connected to the wrist joint base; and
   a jaw base disposed on the wrist joint rotation body;
   the wrist joint assembly further includes a wrist fixed pulley rotatably disposed on the bracket, wherein a distal end of the loop wrist joint driving wire is wound around the wrist joint rotation body, a proximal end is wound around the wrist fixed pulley.
Item 5: the surgical tool according to Item 4, wherein the forceps joint opening-and-closing mechanism further includes:
   at least one forceps rotation body fixedly connected to the first jaw and/or the second jaw and rotatably connected to the jaw base;
   the forceps joint assembly further includes:
      a first set of steering pulleys and a second set of steering pulleys rotatably connected to the jaw base respectively; and
      at least one forceps fixed pulley rotatably disposed on the bracket;
      a distal end of the at least one loop forceps joint driving wire is wound around the at least one forceps rotation bodywhile bypassing the first set of steering pulleys and the second set of steering pulleys, and a proximal end is wound around the at least one forceps fixed pulley.
Item 6: the surgical tool according to Item 5, wherein the at least one forceps rotation body includes:
   a first forceps rotation body and a second forceps rotation body, wherein the first forceps rotation body is fixedly connected to the first jaw and rotatably connected to the jaw base, the second forceps rotation body is fixedly connected to the second jaw and rotatably connected to the jaw base;
   the at least one forceps fixed pulley includes a first forceps fixed pulley and a second forceps fixed pulley rotatably disposed on the bracket respectively;
   the at least one loop forceps joint driving wire includes a first loop forceps joint driving wire and a second loop forceps joint driving wire, a distal end of the first loop forceps joint driving wire is wound around the first forceps rotation bodywhile bypassing the first set of steering pulleys and the second set of steering pulleys, a proximal end is wound around the first forceps fixed pulley, a distal end of the second loop forceps joint driving wire is wound around the second forceps rotation bodywhile bypassing the first set of steering pulleys and the second set of steering pulleys, a proximal end is wound around the second forceps fixed pulley.
Item 7: the surgical tool according to any one of Items 1 to 7, wherein the joint mechanism further includes:
   at least one slider fixedly connected to the at least one loop driving wire; and
   at least one guiding mechanism disposed at a distal end of an arm body along an axial direction of the distal end of the arm body, the slider is slidably connected to the guiding mechanism, the slider is configured to perform linear motion along the guiding mechanism under the driving of the driving rod.
Item 8: the surgical tool according to any one of Items 1 to 7, further comprising at least one transmission mechanism respectively connected to the proximal end of the at least one driving rod, for pushing or pulling the at least one driving rod.
Item 9: The surgical tool according to any one of items 3 to 8, wherein the distal end of the driving rod includes an insulating coating, the insulating coating is configured to insulate the driving rod and the end instrument from each other.
Item 10: the surgical tool according to any one of items 1 to 9,
   the arm body includes a rigid-segment arm body; or
   the arm body includes a deformable arm body.
Item 11: the surgical tool according to Item 10, wherein the deformable arm body includes a continuum arm body, the continuum arm body includes:
   at least one distal continuum segment, the distal continuum segment includes a plurality of distal structural backbones, a distal basal disk, a distal stopping disk, and at least one distal spacer disk disposed between the distal basal disk and the distal stopping disk;
   a distal ends of the plurality of distal structural backbones are fixedly connected to the distal stopping disk, the plurality of distal structural backbones slidably pass through the at least one distal spacer disk and the distal basal disk, the proximal ends of the plurality of distal structural backbones are configured to receive a pushing or pulling drive to drive the motion of the distal continuum segment.
Item 12: a surgical robot system, includes:
   a mobile station, comprising at least one robotic arm; and
   at least one surgical tool according to any one of items 1 to 11, the at least one surgical tool is detachably disposed at the distal end of the robotic arm.

Note that the above are only exemplary embodiments of the present disclosure and the applied technical principles. Those skilled in the art will appreciate that the present disclosure is not limited to specific embodiments herein, and those skilled in the art can make various apparent changes, readjustments and substitutions without departing from the scope of protection of the present disclosure. Thus, although the present disclosure is described in more detail by the above embodiments, the present disclosure is not limited to the above embodiments. Without departing from the concept of the present disclosure, more other equivalent embodiments may be included, and the scope of the present disclosure is determined by the scope of the appended claims.

## Claims

1. A surgical tool comprising:
an arm body including a distal end;
a joint mechanism disposed at a distal end of the arm body; and
at least one driving rod sealingly extending from the distal end of the arm body, wherein the at least one driving rod is connected to the joint mechanism to drive the joint mechanism.

2. The surgical tool according to claim 1, wherein,
the joint mechanism includes at least one joint assembly, wherein the at least one driving rod is connected to the at least one joint assembly respectively, wherein the at least one driving rod extends through the arm body, with a proximal end being configured to receive a pushing or pulling motion so as to drive the at least one joint assembly to open and close, and/or rotate.

3. The surgical tool according to claim 2, wherein the at least one driving rod includes a plurality of driving rods, wherein the at least one joint assembly includes a plurality of joint assemblies, wherein the plurality of driving rods are arranged at intervals, with distal ends sealingly extending from the distal end of the arm body respectively and being connected to the plurality of joint assemblies respectively.

4. The surgical tool according to any one of claims 1 to 3, wherein the driving rod includes a rod body and a sealing member, a distal end of the sealing member is sealingly connected to the distal end of the rod body, and a proximal end of the sealing member is sealingly connected to a distal end of the arm body, so as to isolate at least a portion of the rod body and interior of the arm body from the joint mechanism.

5. The surgical tool according to claim 4, wherein the distal end of the sealing member is sealingly sleeved on the distal end of the rod body, the proximal end seal of the sealing member is disposed at a distal end of the arm body, wherein at least a portion of the sealing member is deformable so as to telescopically move with the motion of the driving rod, wherein the rod body extends through the sealing member.

6. The surgical tool according to claim 5, wherein the sealing member includes an axial portion located at the distal end and a radial portion located at the proximal end, wherein a distal end of the axial portion is sealingly sleeved on the distal end of the driving rod, and the radial portion is sealingly disposed at the distal end of the arm body along the circumferential direction.

7. The surgical tool according to claim 6, wherein,
the axial part of the sealing member is cylindrical, the radial portion of the sealing member includes a flange located at the proximal end, the flange is used for sealingly connecting with the distal end of the arm body.

8. The surgical tool according to any one of claims 1 to 7, further comprising at least one transmission mechanism connected to the proximal end of the at least one driving rod respectively, for pushing or pulling the at least one driving rod.

9. The surgical tool according to any one of claims 2 to 8, wherein the joint assembly includes:
at least one loop driving wire, wherein the at least one loop driving wire forms at least one loop on the at least one joint assembly respectively,
the distal end of the at least one driving rod is respectively connected to at least one loop driving wire of the at least one joint assembly and are configured to push or pull the at least one loop driving wire.

10. The surgical tool according to claim 9, wherein the joint assembly further includes:
a bracket fixedly disposed at the distal end of the arm body;
the at least one joint assembly includes at least one wrist joint assembly, at least one loop driving wire of the wrist joint assembly includes a loop wrist joint driving wire, the at least one driving rod includes a wrist joint driving rod, the wrist joint assembly further includes:
a wrist joint base fixedly disposed at the distal end of the bracket; and
a wrist joint rotation mechanism rotatably disposed on the wrist joint base and connected to the loop wrist joint driving wire, configured to rotate about a first axis when the wrist joint driving rod drives the loop wrist joint driving wire.

11. The surgical tool according to claim 10, wherein the joint mechanism further includes:
an end instrument disposed at the distal end of the joint mechanism, the end instrument includes a first jaw and a second jaw;
the at least one joint assembly includes a forceps joint assembly, wherein the at least one loop driving wire of the forceps joint assembly includes at least one loop forceps joint driving wire, the at least one driving rod includes at least one forceps joint driving rod, wherein the forceps joint assembly further includes:
a forceps joint opening-and-closing mechanism disposed on the wrist joint rotation mechanism and connected to the at least one loop forceps joint driving wire, wherein the first jaw and the second jaw are disposed on the forceps joint opening-and-closing mechanism, the forceps joint opening-and-closing mechanism is configured to rotate together with the wrist joint rotation mechanism and to drive the first jaw and/or the second jaw to open and close about a second axis when the at least one forceps joint driving rod drives the at least one loop forceps joint driving wire.

12. The surgical tool according to claim 11, wherein the wrist joint rotation mechanism includes:
a wrist joint rotation body rotatably connected to the wrist joint base; and
a jaw base disposed on the wrist joint rotation body;
the wrist joint assembly further includes a wrist fixed pulley, which is rotatably disposed on the bracket, wherein a distal end of the loop wrist joint driving wire is wound around the wrist joint rotation body for connection, and a proximal end thereof is wound around the wrist fixed pulley.

13. The surgical tool according to claim 12, wherein the forceps joint opening-and-closing mechanism includes:
at least one forceps rotation body fixedly connected to the first jaw and/or the second jaw and rotatably connected to the jaw base;
the forceps joint assembly further includes:
a first set of steering pulleys and a second set of steering pulleys rotatably connected to the jaw base respectively; and
at least one forceps fixed pulley rotatably disposed on the bracket;
wherein a distal end of the at least one loop forceps joint driving wire is wound around the at least one forceps rotation body, while bypassing the first set of steering pulleys and the second set of steering pulleys, and a proximal end thereof is wound around the at least one forceps fixed pulley.

14. The surgical tool according to claim 13, wherein the at least one forceps rotation body includes:
a first forceps rotation body and a second forceps rotation body, wherein the first forceps rotation body is fixedly connected to the first jaw and is rotatably connected to the jaw base, the second forceps rotation body is fixedly connected to the second jaw and is rotatably connected to the jaw base;
the at least one forceps fixed pulley includes a first forceps fixed pulley and a second forceps fixed pulley fixedly disposed on the bracket respectively;
the at least one loop forceps joint driving wire includes a first loop forceps joint driving wire and a second loop forceps joint driving wire, wherein a distal end of the first loop forceps joint driving wire is wound around the first forceps rotation body while bypassing the first set of steering pulleys and the second set of steering pulleys, a proximal end thereof is wound around the first forceps fixed pulley, wherein a distal end of the second loop forceps joint driving wire is wound around the second forceps rotation bodywhile bypassing the first set of steering pulleys and the second set of steering pulleys, and a proximal end is wound around the second forceps fixed pulley.

15. The surgical tool according to any one of claims 9 to 14, wherein the joint mechanism further includes:
at least one slider fixedly connected to the at least one loop driving wire; and
at least one guiding mechanism disposed at a distal end of the arm body along an axial direction of the distal end of the arm body, wherein the slider is slidably connected to the guiding mechanism and configured to perform linear motion along the guiding mechanism under the driving of the driving rod.

16. The surgical tool according to any one of claims 11 to 15, wherein,
a proximal end of the at least one driving rod is configured to be connected to a monopolar energy generator, wherein the at least one driving rod, at least a portion of the joint mechanism, and the end instrument form a conductive path to perform monopolar operation; or
the at least one forceps joint driving rod includes a first forceps joint driving rod and a second forceps joint driving rod, a proximal ends of the first forceps joint driving rod and the second forceps joint driving rod are configured to be connected to a bipolar energy generator, wherein the first jaw and the second jaw of the distal instrument respectively form a first conductive path and a second conductive path with the first forceps joint driving rod and the second forceps joint driving rod, the first and second conductive paths being insulated from each other, to perform bipolar operation; or
the at least one forceps joint driving rod includes a first forceps joint driving rod and a second forceps joint driving rod, wherein a proximal end of the first forceps joint driving rod is configured to be connected to a monopolar energy generator and a bipolar energy generator, a proximal end of the second forceps joint driving rod is configured to be connected to the bipolar energy generator, the first jaw and the second jaw of the end instrument respectively form a first conductive path and a second conductive path with the first forceps joint driving rod and the second forceps joint driving rod, the first and second conductive paths being insulated from each other, to perform monopolar or bipolar operation.

17. The surgical tool according to any one of claims 11 to 15, wherein,
a distal end of the driving rod includes an insulating coating configured to electrically insulate the driving rod and the end instrument from each other; or
the surgical tool further includes an insulating member disposed on at least a portion of the wrist joint rotation mechanism, or disposed between the wrist joint rotation mechanism and the end instrument, to prevent a non-operating portion of the surgical tool from being energized.

18. The surgical tool according to any one of claims 1 to 17, wherein,
the arm body includes a rigid-segment arm body; or
the arm body includes a deformable arm body.

19. The surgical tool according to claim 18, wherein the deformable arm body includes a continuum arm body, the continuum arm body including:
at least one distal continuum segment, the distal continuum segment includes a plurality of distal structural backbones, a distal basal disk, a distal stopping disk, and at least one distal spacer disk disposed between the distal basal disk and the distal stopping disk;
a distal ends of the plurality of distal structural backbones are fixedly connected to the distal stopping disk, the plurality of distal structural backbones slidably pass through the at least one distal spacer disk and the distal basal disk, the proximal ends of the plurality of distal structural backbones are configured to receive a pushing or pulling drive to drive motion of the distal continuum segment.

20. A surgical robotic system comprising:
a mobile station, comprising at least one robotic arm; and
at least one surgical tool according to any one of claims 1 to 19, wherein the at least one surgical tool is detachably disposed at a distal end of the at least one robotic arm.
